# EUROPEAN PATENT APPLICATION

(11) **EP 4 772 192 A1**
(43) Date of publication of application: **08.07.2026**
(21) Application number: 24858593.7
(22) Date of filing: 28.08.2024
(51) Int. Cl.: A61K 39/395, A61P 3/04, A61P 3/06, A61P 3/10, A61P 9/12, A23L 33/18, C07K 16/28

(54) **USE OF ANTI-IGF-1R ANTIBODY OR ANTIGEN BINDING PORTION THEREOF IN WEIGHT LOSS**

(30) Priority: 01.09.2023 CN 202311122761
(71) Applicant: Suzhou Pro-Heal Pharmaceuticals Technology Co., Ltd., Suzhou, Jiangsu 210000 (CN)
(72) Inventor: LIANG, Xiao, Suzhou, Jiangsu 210000 (CN); GUO, Shuhua, Suzhou, Jiangsu 210000 (CN); MA, Lin, Suzhou, Jiangsu 210000 (CN); PANG, Liang, Suzhou, Jiangsu 210000 (CN)
(74) Representative: Winter, Brandl - Partnerschaft mbB
(86) International application number: PCT/CN2024/114991
(87) International publication number: WO 2025/045059

(57) **Abstract**

The present invention belongs to the field of biomedicine, and specifically relates to the use of anti-IGF-1R antibody or antigen binding portion thereof in weight loss. The present invention provides a use of anti-IGF-1R antibody or antigen binding portion thereof in weight loss. The anti-IGF-1R antibody or antigen binding portion thereof carries out weight loss by means of at least one of the following mechanisms: (1) inhibiting or blocking the binding between IGF-1R and IGF-1; (2) reducing, weakening or neutralizing the activity of IGF-1R; (3) inhibiting cell proliferation; (4) inhibiting fat synthesis and/or accumulation of cells; and (5) inhibiting collagen synthesis of cells. The anti-IGF-1R antibody or the antigen binding portion thereof can significantly inhibit the generation and accumulation of fat, effectively inhibit the formation of collagen, and has a significant effect in treatment of obesity and weight loss.

## Description

The present invention claims priority to the Chinese patent which was filed with the Chinese Patent Office on September 1, 2023 with an application number of 202311122761X, entitled " Use of anti-IGF-1R antibody or antigen binding portion thereof in weight loss ", the entire contents of which are incorporated herein by reference.

### TECHNICAL FIELD

The present invention belongs to the field of biomedicine, and specifically relates to a use of anti-IGF-1R antibody or antigen binding portion thereof in weight loss.

### BACKGROUND OF THE INVENTION

Obesity and complications related to obesity including type 2 diabetes, cardiovascular disease, osteoarthritis, obstructive sleep apnea, and various cancers, are globally one of the main reasons for global disease and death. Obesity refers to excessive weight. Usually, it is overweight when BMI (body mass index ) is of 25kg / m² or above, and it is overweight when BMI is of 30kg / m² or above. Obesity is associated with excessive fat accumulation or abnormal distribution in the body, and abnormal distribution of adipose tissue secretes different adipokines, including leptin, resistin, adiponectin and IL-6, etc. In obese patients, fat tissue releases adipokines which selectively affect the function of tissues and organs and systemic metabolism, leading to conditions such as hypertension, hyperglycemia, high cholesterol, etc. There may even be metal problems such as depression and anxiety, etc.

The basis of obesity management is lifestyle intervention (eating habits and physical exercise). Research shows that the enhanced lifestyle intervention can reduce average body weight by 7-10% in 52 weeks. More and more researchers have recognized that obesity is caused by central appetite disorder, and a long-standing failure to adhere a healthy lifestyle is easy to cause body weight rebound. Therefore, the clinical practice guide suggests a multi-modal treatment method, including drugs and/or surgery, so as to achieve the goal of weight loss ( Bray GA. Evidence-based weight loss intervention: Individual treatment options to maximize patient outcomes. Diabetes Obes Metab. 2021; 23 Suppl 1: 50-62 ). For people who are obese ( BMI ≥ 30 kg / m² ) or overweight ( BMI ≥ 27 kg / m² ) and have at least one of obesity-related diseases ( such as type 2 diabetes, cardiovascular disease and osteoarthritis ), it is recommended to use weight-loss drugs combined with lifestyle interventions. ( Perdomo CM. Contemporary medical, device, and surgical therapies for obesity in adults. Lancet. 2023; 401 (10382): 1116-1130. ) .

At present, clinically used drugs for treating obesity mainly include an early FDA-approved fat absorption inhibitor ( Orlistat and Lorcaserin ), an appetite suppressant ( Phentermine ), an SGLT2 inhibitor ( Naltrexone ), a GLP-1 analog ( Liraglutide), and a PLENITY (Gelesis100) and Imicvree ( Setmelanotide ) approved by FDA in recent years. In addition, dozens of drugs under development for obesity are at different stages of development in clinical trials and preclinical studies. The targets and mechanisms of action are mainly concentrated in the appetite regulation center, GLP-1 receptor, gastrointestinal digestion and absorption, and agonists and inhibitors of other various receptors. Among these, GLP-1 analogs are a relatively good medicine for weight reduction/weight loss. GLP-1 is a gut-derived hormone, which promotes insulin secretion in a glucose-dependent manner by activating a GLP-1 receptor. The mechanism of weight loss of the GLP-1 receptor agonist drug is mainly slowing down the gastrointestinal motility to suppress appetite, which is a relatively good and relatively safe drug for weight loss on the market.

A Chinese patent application 97198413.1 disclosed a derivative of GLP-1 or a GLP-1 analog with up to 6 amino acid changes compared to GLP-1,where the GLP-1 analog has the function of human GLP-1, the derivative has only one lipophilic substituent selected from the group consisting of 8-25 carbon atoms below: CH₃(CH₂)ₙCO-, wherein n is an integer, HOOC(CH₂)ₘCO-, wherein m is an integer, or lithocholic acyl, which is attached to an amino acid residue that is neither an N-terminus nor a C-terminus. Chinese patent application 200480034152.8 disclosed propylene glycol-containing peptide formulations, which relates to preparation method for the formulations and also relates to the formulations for use in treatment of diseases and medical conditions, wherein the peptide is a GLP-1 pro-effect drug selected from GLP-1-amide, GLP-1, GLP-1-amide analog, GLP-1 analog, or the like of any one of these.

The anti-obesity effect of the GLP-1 analog is very significant and stable. However, if it is necessary to achieve a better weight loss effect, generally the dosage of administration is needed to be increased. The large dosage of the GLP-1 derivative easily produces gastrointestinal side effects, which often results in a narrower treatment window due to poor tolerance. In addition, the GLP-1 analog is a polypeptide drug. The chemical and physiological stability of the polypeptide drug are relatively poor, which is particularly easy to be degraded by *in vivo* proteolytic enzymes, resulting in problems such as poor oral absorption, fast metabolism, and short half-life.

Therefore, it is urgent to develop safer and long-acting drugs for the treatment of obesity or for weight loss.

### SUMMARY OF THE INVENTION

Fat is one of the primary tissues of energy storage and metabolism, which can help maintain body energy balance and steady state. However, when the energy intake is too much or insufficient, the adipocytes gradually become larger and increase in number, resulting in body fatlevel exceeding the normal range, thereby initiating obesity. In obesity patients, both the number and size of adipocytes will increase. These adipocytes secrete a series of cytokines and hormones, such as TNF-α, IL-6, PAI-1, etc., which affect whole body metabolism. Therefore, inhibiting excessive differentiation of adipocytes, inhibiting excessive synthesis of fat and reducing excessive fat accumulation are very important for weight loss and control of the progression of obesity.

The primary function of insulin-like growth factor 1 receptor ( IGF-1R ) is to mediate the signal transduction of insulin-like growth factor 1 ( IGF-1 ), thereby regulating various biological processes such as cell proliferation, differentiation and survival. Studies have shown that the activity of IGF-1R can affect the process of metabolism and fat synthesis. The function of IGF-1R in metabolism and fat synthesis is of complexity, which can regulate glucose and lipid metabolism through a variety of mechanisms. The activity level of IGF-1R needs to be maintained within an appropriate range to ensure normal body metabolism and fat synthesis functionality. The over-activation of IGF-1R is closely related to overweight and obesity diseases. IGF-1R over-activation can lead to fat synthesis and energy balance disorders, the mechanism of which is based on the following points: 1. Promoting adipocyte differentiation and fat synthesis. IGF-1R activation can promote adipocyte differentiation and proliferation, and stimulate the synthesis of fatty acids and triglycerides, resulting in increased adipose tissue. 2. Inhibiting fat decomposition metabolism. IGF-1R activation can also inhibit fat decomposition metabolism and reduce the rate of fatty acid oxidation, thereby allowing fatty acids to accumulate in adipocytes. 3. Interfering insulin signaling pathway. IGF-1R has a similar structure and signal pathway with insulin receptors. Over-activation of IGF-1R may interfere with the normal function of the insulin signaling pathway, resulting in insulin resistance, abnormal sugar metabolism and increased fat synthesis. 4. Promoting the synthesis of collagen by fibroblasts in adipose tissue. According to some literature reports, collagen VI may have a functional obstacle on metabolism of adipose tissue. A downstream signal of IGF-1R can effectively stimulate collagen expression, and the over-activated IGF-1R can strongly promote the synthesis of collagen by fibroblasts in adipose tissue.

Anti-IGF-1R antibodies have been successful in multiple treatment fields, such as thyroid eye diseases, etc. However, the anti-IGF-1R antibody has not been successfully applied to the field of treatment of obesity or weight reduction.

In view of the deficiencies of the prior art, the present invention provides a use of anti-IGF-1R antibody or antigen binding portion thereof in weight loss.

The technical solutions proposed in the present invention are as follows:
in one aspect, the present invention provides a use of anti-IGF-1R antibody or antigen binding portion thereof in weight loss.

In an embodiment, the weight loss is induced by the anti-IGF-1R antibody or antigen binding portion thereof via at least one of following mechanisms:
(1) inhibiting or blocking binding between IGF-1R and IGF-1;
(2) reducing, weakening or neutralizing IGF-1R activity;
(3) inhibiting cell proliferation;
(4) inhibiting the fat synthesis and/or accumulation of cells;
(5) inhibiting collagen synthesis of cells.

In an embodiment, the cells comprise fibroblasts.

In another aspect, the present invention provides use of an anti-IGF-1R antibody or antigen binding portion thereof in the preparation of a product for preventing, treating obesity or obesity-related diseases.

In an embodiment, the weight loss is induced by the product via at least one of the following mechanisms:
(1) inhibiting or blocking binding between IGF-1R and IGF-1;
(2) reducing, weakening or neutralizing IGF-1R activity;
(3) inhibiting cell proliferation;
(4) inhibiting the fat synthesis and/or accumulation of cells;
(5) inhibiting collagen synthesis of cells.

In an embodiment, the cells comprise fibroblasts.

In an embodiment, the antibody in both aspects described above may be a monoclonal antibody, such as a humanized antibody.

In an embodiment, the antibody or antigen binding portion thereof in both aspects comprises a light chain variable region and a heavy chain variable region.

In a preferred embodiment, the light chain variable region has:
LCDR1 as shown in SEQ ID NO : 25, SEQ ID NO : 31, SEQ ID NO : 37, SEQ ID NO : 43, SEQ ID NO : 49, SEQ ID NO : 55, SEQ ID NO : 61, SEQ ID NO : 67, SEQ ID NO : 73, SEQ ID NO: 79, SEQ ID NO : 85 or SEQ ID NO: 91 ; and
LCDR2 as shown in SEQ ID NO : 26, SEQ ID NO : 32, SEQ ID NO : 38, SEQ ID NO : 44, SEQ ID NO : 50, SEQ ID NO : 56, SEQ ID NO : 62, SEQ ID NO : 68, SEQ ID NO : 74, SEQ ID NO : 80, SEQ ID NO : 86 or SEQ ID NO : 92 ; and
LCDR3 as shown in SEQ ID NO : 27, SEQ ID NO : 33, SEQ ID NO : 39, SEQ ID NO : 45, SEQ ID NO : 51, SEQ ID NO : 57, SEQ ID NO : 63, SEQ ID NO : 69, SEQ ID NO : 75, SEQ ID NO: 81, SEQ ID NO : 87 or SEQ ID NO: 93.

In a preferred embodiment, the heavy chain variable region has:
HCDR1 as shown in SEQ ID NO : 28, SEQ ID NO : 34, SEQ ID NO : 40, SEQ ID NO : 46, SEQ ID NO : 52, SEQ ID NO : 58, SEQ ID NO : 64, SEQ ID NO : 70, SEQ ID NO : 76, SEQ ID NO : 82, SEQ ID NO : 88 or SEQ ID NO : 94 ; and
HCDR2 as shown in SEQ ID NO : 29, SEQ ID NO : 35, SEQ ID NO : 41, SEQ ID NO : 47, SEQ ID NO : 53, SEQ ID NO : 59, SEQ ID NO : 65, SEQ ID NO : 71, SEQ ID NO : 77, SEQ ID NO : 83, SEQ ID NO : 89 or SEQ ID NO : 95 ; and
HCDR3 as shown in SEQ ID NO : 30, SEQ ID NO : 36, SEQ ID NO : 42, SEQ ID NO : 48, SEQ ID NO : 54, SEQ ID NO : 60, SEQ ID NO : 66, SEQ ID NO : 72, SEQ ID NO : 78, SEQ ID NO : 84, SEQ ID NO : 90 or SEQ ID NO : 96.

In an embodiment, the antibody or antigen binding portion thereof may comprise a heavy chain constant region and/or a light chain constant region.

In an embodiment, the heavy chain constant region may be IgG1, IgG2, IgG3, or IgG4 heavy chain constant regions.

In a preferred embodiment, the light chain of the antibody or the antigen binding portion is formed by splicing a light chain variable region and a light chain constant region as shown in SEQ ID NO : 97; and the heavy chain of the antibody or the antigen binding portion is formed by splicing a heavy chain variable region and a heavy chain constant region as shown in any one of SEQ ID NO : 98-104.

In a preferred embodiment, the heavy chain constant region may be a human IgG1-LALA constant region as shown in SEQ ID NO : 102, which can reduce or eliminate ADCC effects on IGF-1R ⁺ cells.

In a preferred embodiment, the heavy chain constant region may be a human IgG1-YTE constant region shown in SEQ ID NO : 103, which can improve the binding affinity of the antibody to FcRn and prolong the serum half-life thereof.

In a preferred embodiment, the light chain constant region may be a human κ constant region as shown in SEQ ID NO : 97.

In a preferred embodiment, the N-terminal of the heavy chain constant region may be connected to the C-terminal of the heavy chain variable region, and the N-terminal of the light chain constant region may be connected to the C-terminal of the light chain variable region.

In an embodiment, the light chain variable region has:
an amino acid sequence as shown in SEQ ID NO : 1, SEQ ID NO : 3, SEQ ID NO : 5, SEQ ID NO : 7, SEQ ID NO : 9, SEQ ID NO : 11, SEQ ID NO: 13, SEQ ID NO: 15, SEQ ID NO : 17, SEQ ID NO: 19, SEQ ID NO : 21 or SEQ ID NO : 23.

In an embodiment, the heavy chain variable region has:
an amino acid sequence as shown in SEQ ID NO : 2, SEQ ID NO : 4, SEQ ID NO : 6, SEQ ID NO : 8, SEQ ID NO : 10, SEQ ID NO : 12, SEQ ID NO : 14, SEQ ID NO : 16, SEQ ID NO : 18, SEQ ID NO : 20, SEQ ID NO : 22 or SEQ ID NO : 24.

In a preferred embodiment, the light chain variable region has the LCDR1 as shown in SEQ ID NO : 25, the LCDR2 as shown in SEQ ID NO : 26, and the LCDR3 as shown in SEQ ID NO : 27.

In a preferred embodiment, the light chain variable region has the LCDR1 as shown in SEQ ID NO : 31, the LCDR2 as shown in SEQ ID NO : 32, and the LCDR3 as shown in SEQ ID NO : 33.

In a preferred embodiment, the light chain variable region has the LCDR1 as shown in SEQ ID NO : 37, the LCDR2 as shown in SEQ ID NO : 38, and the LCDR3 as shown in SEQ ID NO : 39.

In a preferred embodiment, the light chain variable region has the LCDR1 as shown in SEQ ID NO : 43, the LCDR2 as shown in SEQ ID NO : 44, and the LCDR3 as shown in SEQ ID NO : 45.

In a preferred embodiment, the light chain variable region has the LCDR1 as shown in SEQ ID NO : 49, the LCDR2 as shown in SEQ ID NO : 50, and the LCDR3 as shown in SEQ ID NO : 51.

In a preferred embodiment, the light chain variable region has the LCDR1 as shown in SEQ ID NO : 55, the LCDR2 as shown in SEQ ID NO : 56, and the LCDR3 as shown in SEQ ID NO : 57.

In a preferred embodiment, the light chain variable region has the LCDR1 as shown in SEQ ID NO : 61, the LCDR2 as shown in SEQ ID NO : 62, and the LCDR3 as shown in SEQ ID NO : 63.

In a preferred embodiment, the light chain variable region has the LCDR1 as shown in SEQ ID NO : 67, the LCDR2 as shown in SEQ ID NO : 68, and the LCDR3 as shown in SEQ ID NO : 69.

In a preferred embodiment, the light chain variable region has the LCDR1 as shown in SEQ ID NO : 73, the LCDR2 as shown in SEQ ID NO : 74, and the LCDR3 as shown in SEQ ID NO : 75.

In a preferred embodiment, the light chain variable region has the LCDR1 as shown in SEQ ID NO : 79, the LCDR2 as shown in SEQ ID NO : 80, and the LCDR3 as shown in SEQ ID NO : 81.

In a preferred embodiment, the light chain variable region has the LCDR1 as shown in SEQ ID NO : 85, the LCDR2 as shown in SEQ ID NO : 86, and the LCDR3 as shown in SEQ ID NO : 87.

In a preferred embodiment, the light chain variable region has the LCDR1 as shown in SEQ ID NO : 91, the LCDR2 as shown in SEQ ID NO : 92, and the LCDR3 as shown in SEQ ID NO : 93.

In a preferred embodiment, the heavy chain variable region has the HCDR1 as shown in SEQ ID NO : 28, the HCDR2 as shown in SEQ ID NO : 29, and the HCDR3 as shown in SEQ ID NO : 30.

In a preferred embodiment, the heavy chain variable region has the HCDR1 as shown in SEQ ID NO : 34, the HCDR2 as shown in SEQ ID NO : 35, and the HCDR3 as shown in SEQ ID NO : 36.

In a preferred embodiment, the heavy chain variable region has the HCDR1 as shown in SEQ ID NO : 40, the HCDR2 as shown in SEQ ID NO : 41, and the HCDR3 as shown in SEQ ID NO : 42.

In a preferred embodiment, the heavy chain variable region has the HCDR1 as shown in SEQ ID NO : 46, the HCDR2 as shown in SEQ ID NO : 47, and the HCDR3 as shown in SEQ ID NO : 48.

In a preferred embodiment, the heavy chain variable region has the HCDR1 as shown in SEQ ID NO : 52, the HCDR2 as shown in SEQ ID NO : 53, and the HCDR3 as shown in SEQ ID NO : 54.

In a preferred embodiment, the heavy chain variable region has the HCDR1 as shown in SEQ ID NO : 58, the HCDR2 as shown in SEQ ID NO : 59, and the HCDR3 as shown in SEQ ID NO : 60.

In a preferred embodiment, the heavy chain variable region has the HCDR1 as shown in SEQ ID NO : 64, the HCDR2 as shown in SEQ ID NO : 65, and the HCDR3 as shown in SEQ ID NO : 66.

In a preferred embodiment, the heavy chain variable region has the HCDR1 as shown in SEQ ID NO : 70, the HCDR2 as shown in SEQ ID NO : 71, and the HCDR3 as shown in SEQ ID NO : 72.

In a preferred embodiment, the heavy chain variable region has the HCDR1 as shown in SEQ ID NO : 76, the HCDR2 as shown in SEQ ID NO : 77, and the HCDR3 as shown in SEQ ID NO : 78.

In a preferred embodiment, the heavy chain variable region has the HCDR1 as shown in SEQ ID NO : 82, the HCDR2 as shown in SEQ ID NO : 83, and the HCDR3 as shown in SEQ ID NO : 84.

In a preferred embodiment, the heavy chain variable region has the HCDR1 as shown in SEQ ID NO : 88, the HCDR2 as shown in SEQ ID NO : 89, and the HCDR3 as shown in SEQ ID NO : 90.

In a preferred embodiment, the heavy chain variable region has the HCDR1 as shown in SEQ ID NO : 94, the HCDR2 as shown in SEQ ID NO : 95, and the HCDR3 as shown in SEQ ID NO : 96.

In an embodiment, the obesity-related diseases comprise liver steatosis, hypertension, hyperglycemia, high cholesterol, and the like.

In an embodiment, the product comprises medicine, health care products and food, etc.

In a preferred embodiment, the product comprises the antibody or the antigen binding portion, or a biological material comprising the antibody or the antigen binding portion or a gene thereof, and at least one pharmaceutically acceptable excipient.

The biological material comprises expression vectors and host cells.

In a preferred embodiment, the excipient is selected from solvents, diluents, disintegrants, precipitation inhibitors, surfactants, glidants, binders, lubricants, dispersants, suspending agents, isotonic agents, thickeners, emulsifiers, preservatives, stabilizers, hydrating agents, emulsifying accelerators, buffers, absorbers, colorants, scents, sweeteners, ion exchangers, release agents, coating agents, flavoring agents or antioxidants.

In the present invention, unless otherwise specified, dosage forms are not particularly limited, and dosage forms such as injection, oral liquid, tablet, capsule, dripping pill, spray, inhalant, etc., can be prepared by conventional methods. The dosage form should match the method of the administration of a drug.

The anti-IGF-1R antibody or the antigen binding portion thereof provided by the present invention can significantly inhibit the generation and accumulation of fat, effectively inhibit collagen formation and have a significant effect in treating obesity and in weight loss. The body weight change rate can reach over 10%, and the fat change rate can reach up to about 40%.

### BRIEF DESCRIPTION OF THE DRAWINGS

FIG. 1 shows the inhibition efficiency of anti-IGF-1R antibody on the proliferation of 3T3 cells stably expressing human IGF-1R.
FIG. 2 shows the effect of anti-IGF-1 R antibodies on fat generation and accumulation of fibroblasts by Oil Red O staining, wherein A is a lipid-forming induction group, B is a lipid-forming induction + IGF-1 group, and C is a lipid-forming induction + IGF-1 + AB1 group.
FIG. 3 shows the effect of anti-IGF-1 R antibodies on fat generation and accumulation of fibroblasts by fat generation detection.
FIG. 4 shows the effect of anti-IGF-1R antibody on collagen production of fibroblasts via WesternBlot experiments.
FIG. 5 shows the effect of anti-IGF-1R antibody on body weight of normal male mice (rats).
FIG. 6 shows the effect of anti-IGF-1R antibody on the body weight of normal female mice ( rats ).
FIG. 7 shows the effect of anti-IGF-1R antibody AB1 on body weight of obese mice.
FIG. 8 shows the weight change rate of obese mice after application of anti-IGF-1R antibody AB1, * p < 0.05, ** p < 0.01 ( compared to 0 mpk group).
FIG. 9 shows the total fat change rate of obese mice after administration of anti-IGF-1R antibody AB1, ** p < 0.01 ( compared to 0 mpk group).
FIG. 10 shows the effect of anti-IGF-1R antibody AB9 on body weight of obese mice.
FIG. 11 shows the body weight change rate of obese mice after administration of anti-IGF-1R antibody AB9, * p < 0.05, ** p < 0.01 ( compared to 0 mpk group).
FIG. 12 shows the total fat change of obese mice after administration of anti-IGF-1R antibody AB9, * p < 0.05, ** p < 0.01 ( compared to 0 mpk group).

### DESCRIPTION OF THE PREFERRED EMBODIMENT

Unless otherwise defined, all technical and scientific terms used herein have the same meaning as commonly used in the art to which this invention belongs. For purposes of explaining the present description, the following definitions will be applied and, where appropriate, the terms used in the singular will also include the plural forms, and vice versa.

The expressions " a " and " one " as used herein include plural references unless the context clearly dictates otherwise. For example, reference to " one cell " includes a plurality of such cells and equivalents known to those skilled in the art and the like.

As used herein, the term " about " indicates a range of ± 20% of a value thereafter. In some embodiments, the term " about " indicates a range of ± 10% of the numerical value thereafter. In some embodiments, the term " about " indicates a range of ± 5% of a value thereafter.

The term " IGF-1R " as used herein refers to an insulin-like growth factor 1 receptor. The term comprises variants, homologs, orthologs, and paralogs. For example, antibodies specific to human IGF-1R may cross-react with another species, such as a monkey IGF-1R protein. In other embodiments, the human IGF-1R protein-specific antibody can be completely specific to human IGF-1R protein without cross-reacting with proteins of other species or other types, or can cross-react with IGF-1R proteins of some other species instead of all other species.

As used herein, the term " amino acid " refers to both naturally occurring and synthetic amino acids, as well as amino acid analogs and amino acid mimetics that function in a manner similar to naturally occurring amino acids. The naturally occurring amino acids are amino acids encoded by the genetic code, and those amino acids which are modified, such as hydroxyproline, γ-carboxyglutamic acid, and O-phosphoserine. Amino acid analogs refer to compounds with the same basic chemical structure as the naturally occurring amino acids, i.e., carbon bonded to hydrogen, carboxyl, amino, and R groups, such as homoserine, n-leucine, methionine sulfoxide and methionine methylsulfonium. There are modified R groups ( e.g. positive leucine ) or modified peptide backbones on these analogs, but the same basic chemical structure as naturally occurring amino acids is retained. An amino acid mimetic refers to a compound having a structure different from the general chemical structure of an amino acid but functioning in a manner similar to naturally occurring amino acids.

The term " antibody " as used herein refers to glycoproteins comprising heavy chains ( H ) and light chains ( L ) interconnected by disulfide bonds ( S-S ). Each heavy chain consists of a heavy chain variable region ( abbreviated herein as VH ) and a heavy chain constant region ( abbreviated herein as CH ). The heavy chain constant region consists of three domains CH1, CH2 and CH3. Each light chain consists of a light chain variable region ( abbreviated herein as VL ) and a light chain constant region ( abbreviated herein as VH ). The light chain constant region is composed of one domain CL. The light chain is divided into two categories : a kappa light chain and a lambda light chain ( for example, the light chain constant region Cκ / λ in the present invention indicates that the light chain constant region is a kappa light chain or a lambda light chain). The VH region and the VL region may be further divided into hypervariable regions ( also referred to as complementarity determining regions ( CDRs )) with a conservative framework region or framework region ( FR ) interposed therebetween. Each VH and VL consists of three CDRs and four FRs, arranged in the following order from the amino terminus to the carboxy terminus : FR1, CDR1, FR2, CDR2, FR3, CDR3, FR4. Antibodies comprising monospecific antibodies, bispecific antibodies, and multi-specific antibodies as long as they exhibit the desired biological activity or functionality.

The term " anti-IGF-1R antibody " or "IGF-1R antibody " as used herein refers to antibodies that specifically bind to IGF-1R, and partially or fully neutralize, inhibit or weaken IGF-1R activity, and / or deactivate IGF-1R, prevent IGF-1R response or IGF-1R mediated downstream pathways or other functions which IGF-1R could mediate.

As used herein, the terms "antibody fragment ", "antigen binding fragment ", "antigen binding portion " or similar terms refer to a fragment that remains bound to a target antigen ( e.g. IGF1-R ) in an antibody and has the same activity as the full length of the antibody. Such fragments include, for example, single chain antibodies, single chain Fv fragments ( scFv ), Fd fragments, Fab fragments, Fab ' fragments, or F ( ab ' ) 2 fragments. The ScFv fragment is a single polypeptide chain comprising heavy chain variable regions and light chain variable regions of antibodies derived as scFv-derived antibodies.

As used herein, the term " variable region " or " variable domain " refers to a domain of an antibody that participates in an antigen binding molecule binding to an antigen or a light chain. Variable domains ( VH and VL, respectively ) of heavy and light chains of natural antibodies generally have similar structures, wherein each domain contains four conserved framework regions ( FR ) and three hypervariable regions or super-hypervariable regions ( HVR , also referred to as complementarity determining region CDRs ). A single VH or VL domain may be sufficient to impart antigen-binding specificity.

The terms " hypervariable region ", "super-hypervariable region ", " complementarity determining region ", " HVR "or " CDR ", as used herein, refer to the region of a loop (" hypervariable ring ") that is highly variable and/or structurally defined in the variable domain region of the antibody. Typically, the natural quadruplex antibody comprises six HVRs or CDRs: three ( H1, H2, H3 ) presented in the VH, and three in the VL (L1, L2, L3 ) . Based on Chothia definition rules, exemplary CDRs ( LCDR1, LCDR2, LCDR3, HCDR1, HCDR2, and HCDR3 ) are located at amino acid residues L26-L32 ( L1 ) , L50-L52 ( L2 ) , L91-L96 ( L3 ) , H26-H32 ( H1 ) , H52-H56 ( H2 ) , and H96-H101 ( H3 ) (Chothia et al. J Mol. Biol. 196: 901-917(1987)). Based on Kabat definition rules, exemplary CDRs ( LCDR1, LCDR2, LCDR3, HCDR1, HCDR2, and HCDR3 ) are located at amino acid residues L24-L34 ( L1 ), L50-L56 ( L2 ), L89-L97 ( L3 ), H31-H35 ( H1 ), H50-H65 ( H2 ), and H95-H102 ( H3 ) (Kabat et al. Sequences of Proteins of Immunological Interest, the fifth edition, Public Health Service, National Institutes of Health, Bethesda, MD ( 1991 )). Based on IMGT definition rules, exemplary CDRs ( LCDR1, LCDR2, LCDR3, HCDR1, HCDR2, and HCDR3 ) are located at amino acid residues L27-L32 ( L1 ), L50-L51 ( L2 ), L89-L97 ( L3 ), H26-H33 ( H1 ), H51-H56 ( H2 ) , and H93-H102 ( H3 ) ( Honjo, T. and Alt, F W ( 1995 ) Immunoglobulin genes. Academic Press pp. 3-443). It is well known to those skilled in the art that CDRs of an antibody can be defined in the art by a variety of methods, such as Kabat definition rules based on sequence variability, Chothia definition rules based on structural loop region locations, and reference tools based on CDR transplantation-based antibody humanized designs ( see J Mol Biol. 273: 927-48, 1997 ). It will be appreciated by those skilled in the art that the terms " CDR " and " complementary determining region " of a given antibody or region thereof ( e.g. a variable region ) should be understood to encompass complementary determining regions as defined by any of the above-described known solutions described herein, unless otherwise specified. Although the scope of protection of the present disclosure is based on the sequence shown by Kabat definition rules, the amino acid sequences corresponding to other CDR definition rules should also fall within the protection scope of the present invention.

As used herein, a " framework region " or " FR " refers to a variable domain residue other than a hypervariable region ( HVR ) residue. The variable domain FR is typically composed of four FR domains : FR1, FR2, FR3, and FR4, so that the HVR and FR sequences typically appear in VH ( or VL ) in the following sequence : FR1-H1 ( L1 )-FR2-H2 ( L2 )-FR3-H3 ( L3 )-FR4.

The terms " expression vector ", " expression construct ", " recombinant vector " or " recombinant expression vector " as used herein means that when the isolated nucleic acid molecule encoding the anti-IGFR-1 antibody is connected or integrated onto a vector, the nucleic acid sequence can be directly or indirectly connected to the regulatory element on the vector, as long as the regulatory elements are capable of regulating the translation and expression of the nucleic acid molecule, etc. Surely, these regulatory elements may come directly from the carrier itself, or may be exogenous, i.e. not from the carrier itself. That is, the nucleic acid molecule is operably connected to the regulatory element. By " operably connected " herein, it means that connecting an exogenous gene to a carrier such that regulatory elements within the carrier, such as transcriptional regulatory sequences and translation regulatory sequences, etc., are able to exert their intended function of regulating transcription and translation of exogenous genes. Surely, the polynucleotide encoding the heavy chain and the light chain of the antibody can be separately inserted into different carriers, and it is common to insert onto the same carrier. The common carrier may be, for example, a plasmid, a phage, or the like.

The terms " cell ", " host cell ", or " recombinant cell " as used herein may include an expression vector. The expression vector can be introduced into mammalian cells to obtain recombinant cells, and then these recombinant cells are used to express the antibody or antigen binding portion provided by the present disclosure. By culturing the recombinant cells, corresponding antibodies can be obtained. These available mammalian cells can be, for example, CHO cells, etc.

As used herein, the term " pharmaceutically acceptable carrier, excipient, or adjuvants " should be compatible with the active ingredient, and can be blended with it without reducing or substantially reducing the effect of the drug under normal circumstances. Specific examples of some substances that may be pharmaceutically acceptable carriers or excipients are sugars such as lactose, glucose, and sucrose; starch, such as corn starch and potato starch; cellulose and derivatives thereof, such as sodium methyl cellulose, ethyl cellulose, and methyl cellulose; tragacanth powder; malt; gelatin; talcum; solid lubricants such as stearic acid and magnesium stearate; calcium sulfate; vegetable oils such as peanut oil, cottonseed oil, sesame oil, olive oil, corn oil, and cocoa oil; polyols such as propylene glycol, glycerin, sorbitol, mannitol and polyethylene glycol; alginic acid; emulsifiers such as Tween; wetting agents such as sodium lauryl sulfate; colorants; flavoring agents; tablets, stabilizers; antioxidants; preservatives; pyrogen-free water; isotonic salt solutions; and phosphate buffers, etc. These substances are used as desired to help the stability of the formulation, to help improve activity or its biological effectiveness or to produce an acceptable mouthfeel or odor during oral administration to improve treatment compliance of the patients.

As used herein, the terms " EC50 ", " half effect dose " or " half maximum effect concentration " are antibody concentrations that cause 50% of the maximum effect.

As used herein, the term " IC50 " refers to the half-inhibitory concentration of the antagonist that is measured. It can indicate half of a quantity of a drug or substance ( inhibitor ) in inhibiting certain biological programs ( or certain substances contained in the program, such as enzymes, cell receptors, or microorganisms ).

The term " obesity " as used herein refers to a state of a certain degree of obvious overweight or thickness of fat layer. It is a status that body fat, especially triglyceride accumulates too much. It does not refer to a simple body weight increase, but to a state thatthe body fat tissue excessively accumulates. Changes in body fat due to excessive food intake or body metabolism cause excessive body weight growth and cause human disease, physiological changes, or latency. At present, the body mass index commonly used is referred to as a body mass index ( BMI ), which is also translated into a body mass index. It is an index to calculate weight for height. The specific calculation method is based on the weight ( kilogram , kg) divided by the square of height ( meter , m), that is, BMI = body weight/height/height ( kg/m² ). Based on the analysis between body mass and the degree of obesity, internationally, the Body Mass Index (BMI) established by the World Health Organisation (WHO) is generally used as the benchmark, with a BMI of 25.0-29.9 classified as overweight and 30 or above as obese.

The antibodies or antigen binding portions thereof in the present invention may be incorporated into pharmaceutical compositions suitable for extragastrointestinal administration ( e.g., intravenous, subcutaneous, intraperitoneal, intramuscular ). These pharmaceutical compositions can be prepared in various forms, such as liquids, semi-solids, and solid dosage forms, including but not limited to liquid solutions ( e.g., injection solutions and infusion solutions ), dispersants or suspending agents, tablets, pills, powders, liposomes, and suppositories. A typical pharmaceutical composition is in the form of an injection solution or an infusion solution. The antibody or antigen binding portion thereof of the present invention can be administered by intravenous infusion, injection, intramuscular or subcutaneous injection.

To make the objectives, technical solutions, and advantages of the present invention clearer, the present invention will be further described in detail below with reference to the embodiments. In the embodiments, specific conditions are not indicated, and are carried out according to conventional conditions or conditions suggested by the manufacturer. All reagents or instruments are not indicated by the manufacturer, and are all commercially available conventional products. In order to better illustrate the present invention, numerous specific details are set forth in the following detailed description. The specific embodiments described herein are only used to explain the present invention, and are not intended to constitute any limitation on the present invention. In addition, in the following description, descriptions of well-known structures and techniques are omitted to avoid unnecessarily obscuring the concepts of the present invention. Such structures and techniques are also described in many publications.

### EXAMPLE 1 Molecular screening combining IGF-1R protein

The inventor used recombinant human IGF-1R ( Beijing Baipuersi Biotechnology Co. Ltd. goods number: IGR-H52929 ) as an antigen for 4°C overnight to be coated with a high affinity enzyme label with a coating amount of 50 ng per well, and used 2% BSA ( Beijing Solarbio Technology Co. Ltd. ) for 37°C for 2 hours of closure. The obtained phage library was used and incubated with recombinant human IGF-1R and eluted. After amplification, the phages with binding to recombinant human IGF-1R were enriched. After 3-4 times of repetition, the final phages clones were selected and amplified. According to the phage ELISA detection method, the clones with positive binding to human IGF-1R were obtained. The positive clones were sequenced to obtain the molecules with complete variable region sequences shown in Table 1. Among them, the heavy chain variable region CDR and the light chain variable region CDR of the antibody or its antigen binding portion in the present invention were determined by the Kabat numbering system, and the amino acid sequences of the CDR region are listed in Table 1. As is well known in the art, the heavy chain variable region and the light chain variable region CDR can be determined by, for example, Kabat, Chothia, IMGT, AbM, or Contact numbering systems/methods.

**TABLE 1. CDR sequences of 12 antibodies screened based on Kabat definition rules**

| Antibody number | Light chain CDR1 ( LCDR1 ) | Light chain CDR2 (LCDR2 ) | Light chain CDR3 ( LCDR3 ) | Heavy chain CDR1 ( HCDR1 ) | Heavy chain CDR2 ( HCDR2 ) | Heavy chain CDR3 ( HCDR3 ) |
|---|---|---|---|---|---|---|
| AB1 | SEQ ID NO : 25 : SASSHWHS YMH | SEQ ID NO : 26 : DTINLMT | SEQ ID NO : 27 : QQITGYPSV AE | SEQ ID NO : 28 : GMHWG | SEQ ID NO : 29 : GMHWG | SEQ ID NO : 30 : GSVEPYTH |
| AB2 | SEQ ID NO : 31 : RASQGIRND LG | SEQ ID NO : 32 : AASRLHR | SEQ ID NO : 33 : LQHNSYPCS | SEQ ID NO : 34: SYAMN | SEQ ID NO : 35 : AISGSGGTT FYADSVKG | SEQ ID NO : 36 : DLGWSDSY YYYYGMDV |
| AB3 | SEQ ID NO : 37 : RSSQSIVHS NGNTWLQ | SEQ ID NO : 38 : KVSNRLW | SEQ ID NO : 39 : FQGSHVPW T | SEQ ID NO : 40 : GGWLWN | SEQ ID NO : 41 : WISWDGTN NWKPSLKD | SEQ ID NO : 42 : WGRVFFDW |
| AB4 | SEQ ID NO : 43 : QGDSLRSY YAT | SEQ ID NO : 44 : GENKRPS | SEQ ID NO : 45 : KSRDGSGQ HLV | SEQ ID NO : 46 : SYAIS | SEQ ID NO : 47 : GIIPIFGTAN YAQKFQG | SEQ ID NO : 48 : APLRFLEWS TQDHYYYY YMDV |
| AB5 | SEQ ID NO : 49 : RASQSVSSY LA | SEQ ID NO : 50 : DASKRAT | SEQ ID NO : 51 : QQRSKWPP WT | SEQ ID NO : 52 : SYGMH | SEQ ID NO : 53 : IIWFDGSST YYADSVRG | SEQ ID NO : 54 : ELGRRYFDL |
| AB6 | SEQ ID NO : 55 : RASQSIGSS LH | SEQ ID NO : 56 : YASQSLS | SEQ ID NO : 57 : HQSSRLPHT | SEQ ID NO : 58 : SFAMH | SEQ ID NO : 59 : VIDTRGATY YADSVKG | SEQ ID NO : 60 : LGNFYYGM DV |
| AB7 | SEQ ID NO : 61 : RSSQSIVHS NGNTYLQ | SEQ ID NO : 62 : KVSNRLY | SEQ ID NO : 63 : FQGSHVPW T | SEQ ID NO : 64 : GGYLWN | SEQ ID NO : 65 : YISYDGTNN YKPSLKD | SEQ ID NO : 66 : YGRVFFDY |
| AB8 | SEQ ID NO : 67 : RSSQSIVHS NVNTYLE | SEQ ID NO : 68 : KVSNRFS | SEQ ID NO : 69 : FQGSHVPPT | SEQ ID NO : 70 : SYWMH | SEQ ID NO : 71 : EINPSNGRT NYNQKFQG | SEQ ID NO : 72 : GRPDYYGSS KWYFDV |
| AB9 | SEQ ID NO : 73 : RSSQSLLHS NGYNYLD | SEQ ID NO : 74 : LGSNRAS | SEQ ID NO : 75 : MQGTHWPL T | SEQ ID NO : 76 : SSNWWS | SEQ ID NO : 77 : EIYHSGSTN YNPSLKS | SEQ ID NO : 78 : WTGRTDAF DI |
| AB10 | SEQ ID NO : 79 : SASSHWHS YMH | SEQ ID NO : 80 : DTINLMT | SEQ ID NO : 81 : QQITGYPSV AE | SEQ ID NO : 82 : GMHWN | SEQ ID NO : 83 : YISSGSTDY NPSLKS | SEQ ID NO : 84 : GSVEPYTH |
| AB11 | SEQ ID NO : 85 : KASQEVGT AVA | SEQ ID NO : 86 : WASTRHT | SEQ ID NO : 87 : QQYSNYPLT | SEQ ID NO : 88 : DYYMY | SEQ ID NO : 89 : YITNYGGST YYPDTVKG | SEQ ID NO : 90 : QSNYDYDG WFAY |
| AB12 | SEQ ID NO : 91 : QASRDIRNY LN | SEQ ID NO : 92 : DASSLQT | SEQ ID NO : 93 : QQFDSLPHT | SEQ ID NO : 94 : IYRMQ | SEQ ID NO : 95 : GISPSGGTT WYADSVKG | SEQ ID NO : 96 : WSGGSGYA FDI |

### EXAMPLE 2 Construction and expression of anti-IGF-1R antibody

The light chain variable region ( AB1-12 ) and the light chain constant region ( SEQ ID NO : 97 ) cloned in Example 1 were directly spliced to form a light chain, and the heavy chain variable region ( AB1-12 ) and the heavy chain constant region ( SEQ ID NO : 98 ) cloned in Example 1 were directly spliced to form a heavy chain. The amino acid sequences of light chain / heavy chain variable region and light chain / heavy chain constant region are shown in Table 2 and Table 3.

After codon optimization of the amino acid sequences of the light chain and heavy chain, an expression vector was constructed by inserting the sequence of the human κ / IgG1-LALA constant region ( the amino acid sequences of the light chain constant region and the heavy chain constant region were SEQ ID NO : 97 and 102, respectively ) between the restriction enzyme cutting site EcoRI / HindIII of pTT5 to construct the.

The sequences of the coding variable region and the human IgG1 ( N297A ) / κ constant region ( the amino acid sequences of the heavy chain constant region and the light chain constant region were SEQ ID NO : 33 and 34, respectively ) were inserted into the restriction site XhoI / BamHI of pCDNA3.1 ( Invitrogen, USA ) to construct the expression vector. The obtained expression vector was transfected into HEK-293F cells using PEI. Specifically, HEK-293F cells were cultured in Free StyleTM 293 expression medium ( Gibco, Cat # : 12338-018 ), and each expression vector was transfected into cells by PEI transfection. The ratio of DNA to PEI was 1 : 3, and the amount of DNA added to per milliliter of cell culture medium was 1.5 µg. The transfected HEK-293F cells were cultured at 37 °C in a 5 % CO₂ incubator at 120 rpm. After 10-12 days, the cell culture supernatant was collected. Monoclonal antibodies were enriched and purified by a pre-equilibrated protein-G affinity column ( GE, Cat # : 17040501 ) and eluted with an elution buffer ( 20 mM citric acid, pH 3.0-3.5 ). After that, the antibody was stored in PBS ( pH 7.0 ) and the antibody concentration was detected by NanoDrop.

**TABLE 2. Light chain and heavy chain variable region amino acid sequence**

| Antibody number | Light chain variable area ( VL ) | Heavy chain variable area(VL) |
|---|---|---|
| AB1 | EIVLTQSPATLSLSPGERATLSCSASSHWH SYMHWYQQKPGQAPRLLIYDTINLMTGIP ARFSGSRSGTDYTLTISSLEPEDAAVYYCQ QITGYPSVAEFGQGTKLEIK(SEQ ID NO : 1) | QVQLQESGPGLVKPPGTLSLTCALSGYSV TGMHWGWVRQPPGKGLEWIGYISSGSTD YNPSLKSRVTISSDTSKNQLSLKLSSVTAA DTAVYYCARGSVEPYTHWGQGTLVTVSS( SEQ ID NO : 2) |
| AB2 | DIQMTQFPSSLSASVGDRVTITCRASQGIR NDLGWYQQKPGKAPKRLIYAASRLHRGV PSRFSGSGSGTEFTLTISSLQPEDFATYYCL QHNSYPCSFGQGTKLEIKR(SEQ ID NO : 3) | EVQLLESGGGLVQPGGSLRLSCTASGFTFS SYAMNWVRQAPGKGLEWVSAISGSGGTT FYADSVKGRFTISRDNSRTTLYLQMNSLR AEDTAVYYCAKDLGWSDSYYYYYGMDV WGQGTTVTVSSA(SEQ ID NO : 4) |
| AB3 | DIVMTQSPLSLPVTPGQPASISCRSSQSIVH SNGNTWLQWWLQKPGQSPQLLIWKVSN RLWGVPDRFSGSGSGTDFTLKISRVQAQD VGVWWCFQGSHVPWTFGQGTKVQIKR(S EQ ID NO : 5) | QVQLQQSGPGLVKPSQTLSLTCTVSGWSIS GGWLWNWIRQPPGKGLQWIGWISWDGT NNWKPSLKDRVTISVDTSKNQFSLKLSSV TAADTAVWWCARWGRVFFDWWGQGTL VTVSSA(SEQ ID NO : 6) |
| AB4 | SSELTQDPAVSVALGQTVRITCQGDSLRS YYATWYQQKPGQAPILVIYGENKRPSGIP DRFSGSSSGNTASLTITGAQAEDEADYYC KSRDGSGQHLVFGGGTKLTVLG(SEQ ID NO : 7) | EVQLVQSGAEVKKPGSSVKVSCKASGGTF SSYAISWVRQAPGQGLEWMGGIIPIFGTAN YAQKFQGRVTITADKSTSTAYMELSSLRS EDTAVYYCARAPLRFLEWSTQDHYYYYY MDVWGKGTTVTVSSA(SEQ ID NO : 8) |
| AB5 | EIVLTQSPATLSLSPGERATLSCRASQSVSS YLAWYQQKPGQAPRLLIYDASKRATGIPA RFSGSGSGTDFTLTISSLEPEDFAVYYCQQ RSKWPPWTFGQGTKVESKR(SEQ ID NO : 9) | QVELVESGGGVVQPGRSQRLSCAASGFTF SSYGMHWVRQAPGKGLEWVAIIWFDGSS TYYADSVRGRFTISRDNSKNTLYLQMNSL RAEDTAVYFCARELGRRYFDLWGRGTLV SVSSA(SEQ ID NO : 10) |
| AB6 | EIVLTQSPGTLSVSPGERATLSCRASQSIGS SLHWYQQKPGQAPRLLIKYASQSLSGIPD RFSGSGSGTDFTLTISRLEPEDFAVYYCHQ SSRLPHTFGQGTKVEIKR(SEQ ID NO : 11) | EVQLVQSGGGLVKPGGSLRLSCAASGFTF SSFAMHWVRQAPGKGLEWISVIDTRGAT YYADSVKGRFTISRDNAKNSLYLQMNSLR AEDTAVYYCARLGNFYYGMDVWGQGTT VTVSSA(SEQ ID NO : 12) |
| AB7 | (SEQ ID NO : 13) | (SEQ ID NO : 14) |
| AB8 | DVVMTQTPLSLPVSLGDPASISCRSSQSIV HSNVNTYLEWYLQKPGQSPRLLIYKVSNR FSGVPDRFSGSGAGTDFTLRISRVEAEDLG IYYCFQGSHVPPTFGGGTKLEIKR(SEQ ID NO : 15) | QVQLVQSGAEVVKPGASVKLSCKASGYT FTSYWMHWVKQRPGQGLEWIGEINPSNG RTNYNQKFQGKATLTVDKSSSTAYMQLS SLTSEDSAVYYFARGRPDYYGSSKWYFD VWGQGTTVTVSS(SEQ ID NO : 16) |
| AB9 | DVVMTQSPLSLPVTPGEPASISCRSSQSLL HSNGYNYLDWYLQKPGQSPQLLIYLGSN RASGVPDRFSGSGSGTDFTLKISRVEAED VGVYYCMQGTHWPLTFGQGTKVEIKR(S EQ ID NO : 17) | QVQLQESGPGLVKPSGTLSLTCAVSGGSIS SSNWWSWVRQPPGKGLEWIGEIYHSGST NYNPSLKSRVTISVDKSKNQFSLKLSSVTA ADTAVYYCARWTGRTDAFDIWGQGTMV TVSSA(SEQ ID NO : 18) |
| AB10 | QIVLTQSPAIMSRVVGEKVTMTCSASSHW HSYMHWYQQKPGSSPSLYIYDTINLMTG VPARFSGSRSGTSYSLTIVSMEREDAATY YCQQITGYPSVAEFGAGTKLEIK(SEQ ID NO : 19) | DVQLQESGPELMKPSQIVSLTCKLSGYSVT GMHWNWIRQVSESKLEWIGYISSGSTDYN PSLKSRISITSETSKNQLFLQLNIVTTEDIAT YYCARGSVEPYTHWGQGTLVTVSA(SEQ ID NO : 20) |
| AB11 | DIQLTQSPSSLSASVGDRVTITCKASQEVG TAVAWYQQKPGKAPKLLIYWASTRHTGV PSRFSGSGSGTDFTFTISSLQPEDIATYFCQ QYSNYPLTFGQGTKVEIKR(SEQ ID NO : 21) | QVQLQESGGGVVQPGRSLRLSCSASGFTF SDYYMYWVRQAPGKGLEWVAYITNYGG STYYPDTVKGRFTISRDNAKNTLFLQMDS LRPEDTGVYFCARQSNYDYDGWFAYWG QGTPVTVSS(SEQ ID NO : 22) |
| AB12 | DIQMTQSPLSLSASVGDRVTITCQASRDIR NYLNWYQQKPGKAPKLLIYDASSLQTGV PSRFGGSGSGTDFSFTIGSLQPEDIATYYC QQFDSLPHTFGQGTKLEIK(SEQ ID NO : 23) | EVQLLESGGGLVQPGGSLRLSCAASGFTFS IYRMQWVRQAPGKGLEWVSGISPSGGTT WYADSVKGRFTISRDNSKNTLYLQMNSL RAEDTAVYYCARWSGGSGYAFDIWGQGT MVTVSS(SEQ ID NO : 24) |

**TABLE 3. Amino acid sequence of light chain and heavy chain constant region**

| Constant region | Sequence number | Sequence |
|---|---|---|
| | SEQ ID NO : 98 | |
| Heavy chain constant region | SEQ ID NO : 99 | ASTKGPSVFPLAPCSRSTSESTAALGCLVKDYFPEPVTVSWNSGA LTSGVHTFPAVLQSSGLYSLSSVVTVPSSNFGTQTYTCNVDHKPS NTKVDKTVERKCCVECPPCPAPPVAGPSVFLFPPKPKDTLMISRTP EVTCVVVDVSHEDPEVQFNWYVDGVEVHNAKTKPREEQFNSTF RVVSVLTVVHQDWLNGKEYKCKVSNKGLPAPIEKTISKTKGQPR EPQVYTLPPSREEMTKNQVSLTCLVKGFYPSDIAVEWESNGQPEN NYKTTPPMLDSDGSFFLYSKLTVDKSRWQQGNVFSCSVMHEAL HNHYTQKSLSLSPGK |
| | SEQ ID NO : 100 | ASTKGPSVFPLAPCSRSTSGGTAALGCLVKDYFPEPVTVSWNSGA LTSGVHTFPAVLQSSGLYSLSSVVTVPSSSLGTQTYTCNVNHKPS NTKVDKRVELKTPLGDTTHTCPRCPEPKSCDTPPPCPRCPEPKSC DTPPPCPRCPEPKSCDTPPPCPRCPAPELLGGPSVFLFPPKPKDTLM ISRTPEVTCVVVDVSHEDPEVQFKWYVDGVEVHNAKTKPREEQY NSTFRVVSVLTVLHQDWLNGKEYKCKVSNKALPAPIEKTISKTK GQPREPQVYTLPPSREEMTKNQVSLTCLVKGFYPSDIAVEWESSG QPENNYNTTPPMLDSDGSFFLYSKLTVDKSRWQQGNIFSCSVMH EALHNRFTQKSLSLSPGK |
| | SEQ ID NO : 101 | ASTKGPSVFPLAPCSRSTSESTAALGCLVKDYFPEPVTVSWNSGA LTSGVHTFPAVLQSSGLYSLSSVVTVPSSSLGTKTYTCNVDHKPS NTKVDKRVESKYGPPCPSCPAPEFLGGPSVFLFPPKPKDTLMISRT PEVTCVVVDVSQEDPEVQFNWYVDGVEVHNAKTKPREEQFNST YRVVSVLTVLHQDWLNGKEYKCKVSNKGLPSSIEKTISKAKGQP REPQVYTLPPSQEEMTKNQVSLTCLVKGFYPSDIAVEWESNGQPE NNYKTTPPVLDSDGSFFLYSRLTVDKSRWQEGNVFSCSVMHEAL HNHYTQKSLSLSLGK |
| | SEQ ID NO : 102 | ASTKGPSVFPLAPSSKSTSGGTAALGCLVKDYFPEPVTVSWNSGA LTSGVHTFPAVLQSSGLYSLSSVVTVPSSSLGTQTYICNVNHKPSN TKVDKKVEPKSCDKTHTCPPCPAPEAAGGPSVFLFPPKPKDTLMI SRTPEVTCVVVDVSHEDPEVKFNWYVDGVEVHNAKTKPREEQY NSTYRVVSVLTVLHQDWLNGKEYKCKVSNKALPAPIEKTISKAK GQPREPQVYTLPPSRDELTKNQVSLTCLVKGFYPSDIAVEWESNG QPENNYKTTPPVLDSDGSFFLYSKLTVDKSRWQQGNVFSCSVMH EALHNHYTQKSLSLSPGK |
| | SEQ ID NO : 103 | ASTKGPSVFPLAPSSKSTSGGTAALGCLVKDYFPEPVTVSWNSGA LTSGVHTFPAVLQSSGLYSLSSVVTVPSSSLGTQTYICNVNHKPSN TKVDKKVEPKSCDKTHTCPPCPAPELLGGPSVFLFPPKPKDTLYIT REPEVTCVVVDVSHEDPEVKFNWYVDGVEVHNAKTKPREEQYN STYRVVSVLTVLHQDWLNGKEYKCKVSNKALPAPIEKTISKAKG QPREPQVYTLPPSRDELTKNQVSLTCLVKGFYPSDIAVEWESNGQ PENNYKTTPPVLDSDGSFFLYSKLTVDKSRWQQGNVFSCSVMHE ALHNHYTQKSLSLSPGK |
| | SEQ ID NO : 104 | ASTKGPSVFPLAPSSKSTSGGTAALGCLVKDYFPEPVTVSWNSGA LTSGVHTFPAVLQSSGLYSLSSVVTVPSSSLGTQTYICNVNHKPSN TKVDKKVEPKSCDKTHTCPPCPAPEAAGGPSVFLFPPKPKDTLYI TREPEVTCVVVDVSHEDPEVKFNWYVDGVEVHNAKTKPREEQY NSTYRVVSVLTVLHQDWLNGKEYKCKVSNKALPAPIEKTISKAK GQPREPQVYTLPPSRDELTKNQVSLTCLVKGFYPSDIAVEWESNG QPENNYKTTPPVLDSDGSFFLYSKLTVDKSRWQQGNVFSCSVMH EALHNHYTQKSLSLSPGK |
| Light chain constant region | SEQ ID NO : 97 | |

### EXAMPLE 3 Anti-IGF-1R antibody binding to human IGF-1R

To determine whether the anti-IGF-1R antibody binds to human IGF-1R, inventors used an ELISA saturation concentration method for determination, the principle of which is : determining the affinity of the antibody to IGF-1R, performing gradient dilution on the antibody in the presence of a small amount of IGF-1R to detect the concentration of the antibody / IGF-1R complex. When the concentration of the antibody / IGF-1 R complex accounts for half of the total IGF-1R concentration, the concentration value of the corresponding antibody (EC50) is the KD value of the antibody to IGF-1R.

FACS cell binding assay was performed using HEK293T cells which stably over-expressed human IGF-1R. Briefly, 10⁵ HEK293T cells in 100 µL medium were plated on a 96-well V plate and 50 µL of gradient diluted ( 3-fold diluted ) anti-IGF-1R antibody was added. After incubation at 4 °C for 1 hour, the 96-well plates were washed three times with PBST. Afterwards, 1000-fold diluted PE-goat anti-human IgG ( purchased from BioLegend ) was added. After incubation at 4 °C for 1 hour, the 96-well plate was washed three times with PBS, and then the cell fluorescence was detected by flow cytometry ( purchased from Beckman ) to calculate the EC50 value. The EC50 value of the antibody is shown in Table 4. The results showed that the affinity of different anti-IGF-1R antibodies to HEK293T cells over-expressing human IGF-1R was different.

**TABLE 4. Determination of EC50 values ( equivalent to KD values ) of anti-IGF-1R antibodies in combination with human IGF-1R**

| Antibody number | EC50 ( nM ) |
|---|---|
| AB1 | 0.97 |
| AB2 | 2.52 |
| AB3 | 1.43 |
| AB4 | 0.57 |
| AB5 | 1.22 |
| AB6 | 2.29 |
| AB7 | 0.85 |
| AB8 | 0.21 |
| AB9 | 1.76 |
| AB10 | 3.18 |
| AB11 | 1.62 |
| AB12 | 1.21 |

### EXAMPLE 4 Anti-IGF-1R antibody inhibits human IGF-1R/IGF-1 interaction

HEK293T cells stably over-expressing human IGF-1R in EXAMPLE 3 were used for blocking detection by FACS. Briefly, 10⁵ HEK293T / human IGF-1R cells in 100 µL medium were plated on a 96-well V-plate, and 50 µL of gradient dilution of anti-IGF-1R antibody was added. After incubation at 4 °C for 1 hour, the plates were washed three times with PBST. After incubation, 100 µL 200 µg / mL Biotinylated Human IGF-I Protein ( ACROBiosystems, Cat # : IG1-H82F7 ) was added. After 1 hour of incubation at 4 °C, the plate was washed three times with PBST, and 1000-fold diluted Alexa Fluor ^{®} 488 Streptavidin ( Jackson, Cat # : 016-540-084 ) was added. After incubation at 4 °C for 1 hour, the plate was washed three times with PBST, and the cell fluorescence was detected by flow cytometry ( Beckman ) to calculate the IC50 value. The IC50 values of each antibody are shown in TABLE 5. The results showed that the inhibitory activity of different anti-IGF-1R antibodies on IGF-1R / IGF-1 interaction was different, which was not completely related to the affinity strength in EXAMPLE 3.

**TABLE 5. The IC50 value of anti-IGF-1R antibody inhibiting human IGF-1R/IGF-1**

| Antibody number | IC50 ( nM ) |
|---|---|
| AB1 | 1.72 |
| AB2 | 4.32 |
| AB3 | 2.48 |
| AB4 | 1.03 |
| AB5 | 8.25 |
| AB6 | 6.52 |
| AB7 | 1.84 |
| AB8 | 0.97 |
| AB9 | 4.27 |
| AB10 | 5.31 |
| AB11 | 3.72 |
| AB12 | 2.52 |

### EXAMPLE 5 Evaluation of anti-IGF-1R antibody biological activity

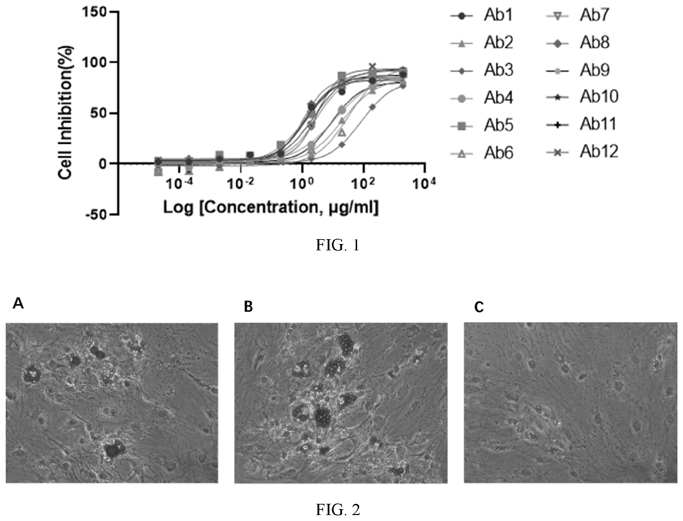

In this study, mouse fibroblast 3T3 was used as the research object to construct 3T3 cells stably over-expressing human IGF-1 R, and the proliferation activity of fibroblasts was used as the biological characteristics to illustrate the biological activity of anti-IGF-1R antibody. To this end, the activity of the anti-IGF-1R antibody in this invention can be evaluated by detecting the cell proliferation of 3T3 / IGF-1R cells. The CCK8 method was used to detect the inhibitory efficiency of anti-IGF-1R antibody on the proliferation of 3T3 / IGF-1R cells ( FIG. 1 ). IC50 values were calculated, and the IC50 values of each antibody are shown in TABLE 6. The results showed that the inhibitory activity of different anti-IGF-1R antibodies on the proliferation of 3T3 / IGF-1R cells was different, which was not completely consistent with the blocking activity shown in EXAMPLE 4.

**TABLE 6. Biological activity of Anti-IGF-1R antibody**

| Antibody number | IC50 ( nM ) |
|---|---|
| AB1 | 8.25 |
| AB2 | 133.27 |
| AB3 | 567.87 |
| AB4 | 89.07 |
| AB5 | 9.72 |
| AB6 | 219.87 |
| AB7 | 19.58 |
| AB8 | 7.60 |
| AB9 | 61.61 |
| AB10 | 16.65 |
| AB11 | 9.11 |
| AB12 | 20.45 |

### EXAMPLE 6 Anti-IGF-1R antibody inhibits fibroblast adipogenesis and lipogenesis accumulation

The IGF-1R signaling pathway plays an important role in cellular adipogenesis and lipogenesis accumulation. In this example, the isolated primary fibroblasts were used as the research object, and the evaluation of the role of the anti-IGF-1R antibody in lipogenesis accumulation was achieved by detecting fat accumulation. Specifically, the isolated primary fibroblasts were cultured in DMEM medium with 10 % FBS, and the fibroblasts were induced to differentiate into adipocytes by fat induction medium. During the induction of differentiation, IGF-1 was added alone and added with anti-IGF-1R antibodies, separately. The formation of adipocytes was observed by oil red staining. The results showed that anti-IGF-1R antibody could significantly inhibit the formation and accumulation of fat ( FIG. 2 ).

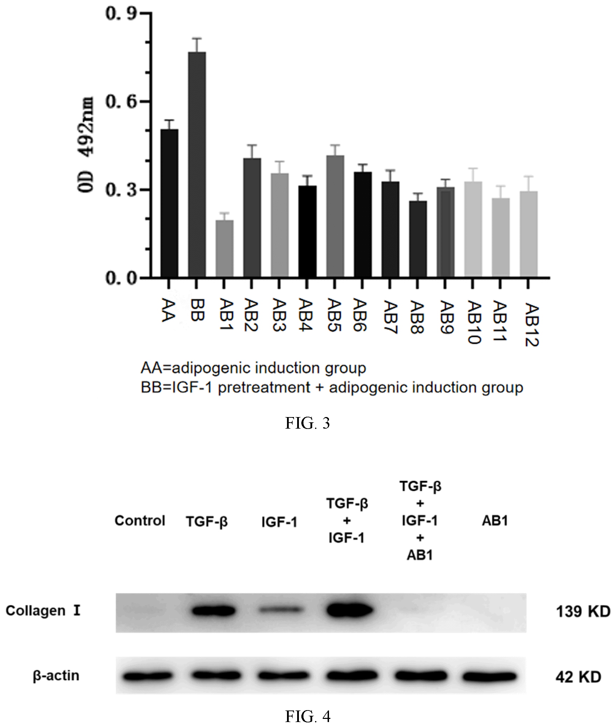

The 3T3-huIGF-1R cell suspension over-expressing human IGF-1R was inoculated in a 6-well plate and treated with a self-made adipogenic induction solution to induce adipogenic differentiation. The adipogenic induction medium was DMEM + 10 % fetal bovine serum + 1 % P / S + 0.5mM IBMX + 0.25µM dexamethasone + 1µg / mL insulin. The control group and the experimental group were set as follows : adipogenic induction group, IGF-1 pretreatment + adipogenic induction group, IGF-1 pretreatment + AB1-12 + adipogenic induction group. The cells were fixed with 3.5 % formaldehyde solution for 10 minutes, rinsed with PBS twice, and incubated with 1.5 % oil red O staining solution in 60 % isopropanol for 1 h. The cells stained with oil red O were washed with PBS to remove the excess dye ( washed 6 times ), and the fat droplets were dissolved in the extract. The absorbance at 492 nm was read by a microplate reader. The results are shown in FIG. 3. The results showed that 3T3 cells over-expressing human IGF-1R showed a certain degree of adipogenesis and accumulation after induction by adipogenic induction medium. After IGF-1 pretreatment and adipogenic induction, the degree of adipogenesis and accumulation in 3T3 cells was the highest. After adding AB1-12 at the same time, the adipogenesis and accumulation of 3T3 cells were significantly inhibited to varying degrees. Therefore, different anti-IGF-1R antibodies ( AB1-AB12 ) in this invention can significantly inhibit the production and accumulation of fat.

### EXAMPLE 7 Anti-IGF-1R antibody inhibits fibroblast collagen

According to some literature reports, collagen VI may cause dysfunction in the metabolism of adipose tissue. In this example, the isolated primary fibroblasts were used as the research object, and the role of anti-IGF-1R antibody in collagen production was evaluated by detecting the expression of collagen. Specifically, the isolated primary fibroblasts were cultured in DMEM medium with 10 % FBS, and adipocyte differentiation was induced by fat-inducing medium. During the culture process, TGF-β, IGF-1 and IGF-1 and TGF-β were added separately to induce collagen expression. Anti-IGF-1R antibody was added during the induction process. The expression of collagen I was detected by immunoblotting after SDS-PAGE after the cellular protein was extracted, and the results are shown in FIG. 4. The results showed that the anti-IGF-1R antibody provided by the invention effectively inhibited the formation of collagen. In more tests, the anti-IGF-1R antibody provided by the invention can effectively inhibit the production of collagen.

### EXAMPLE 8 Effect of body weight control by anti-IGF-1R antibody in rats

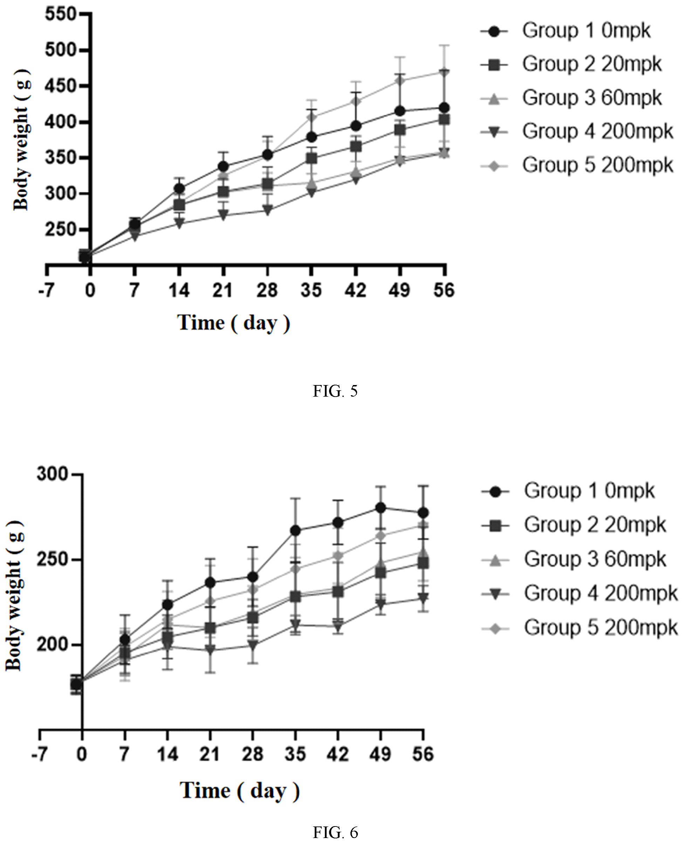

In this example, 50 SD rats ( Beijing Huafukang Biotechnology Co., Ltd. ) were randomly assigned to 5 groups, 10 rats in each group. After grouping, the average body weight of each group of rats was not statistically different at the 5.0% test level. The antibody molecule was AB1, and the test doses were set to 20 , 60 and 200 mg / kg. In addition, a subcutaneous control group was set up, and the dose was consistent with the dose of the intravenous high-dose group. At the same time, the excipient control group was set up, and the excipient reference substance was given intravenously. The information of each experimental group is as follows, Group 1 : excipient control group, intravenous administration ; Group 2 : 20 mpk ( Milligrams Per Kilograms ) AB1, intravenous administration ; Group 3 : 60 mpk AB1, intravenous administration ; Group 4 : 200 mpk AB1, intravenous administration ; Group 5 : 200 mpk AB1, subcutaneous administration. Before and after administration ( weighing 8 times in total ), the body weight of rats was weighed once a week, and the body weight of male and female rats was counted respectively. The results showed that compared with the control group, the weight gain of the intravenous administration group was inhibited ; with the increase of the dose of administration, the inhibition of body weight gain exhibited concentration dependence to some extent( FIG.5 and FIG.6 ).

### EXAMPLE 9 Effect of anti-IGF-1R antibody in diet-induced mouse obesity model

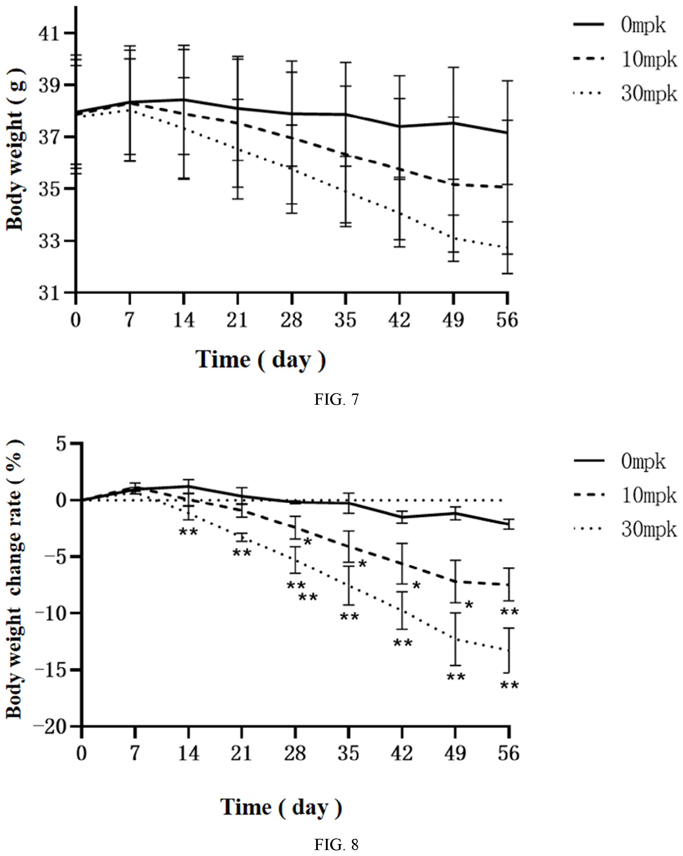

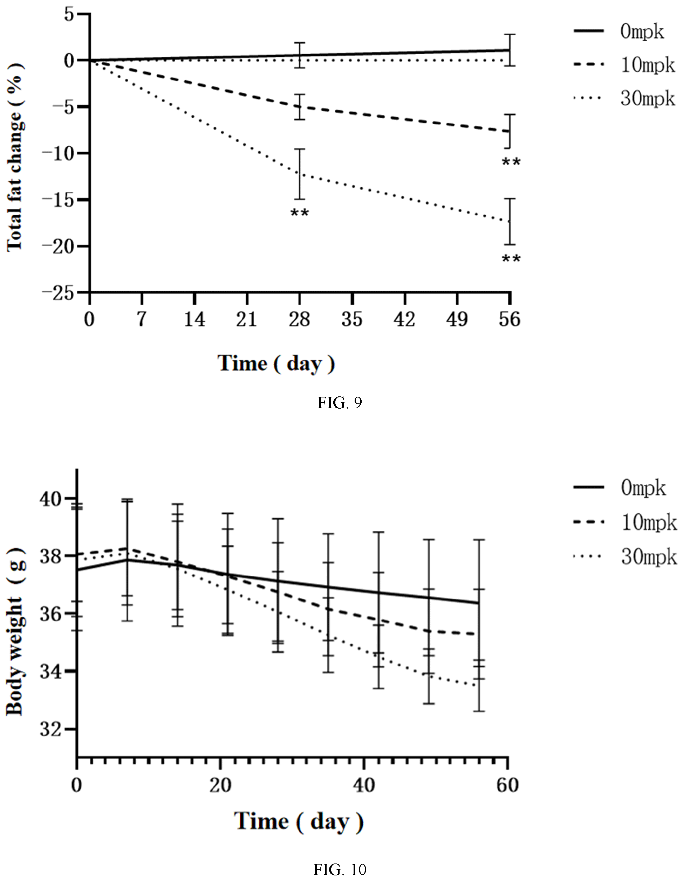

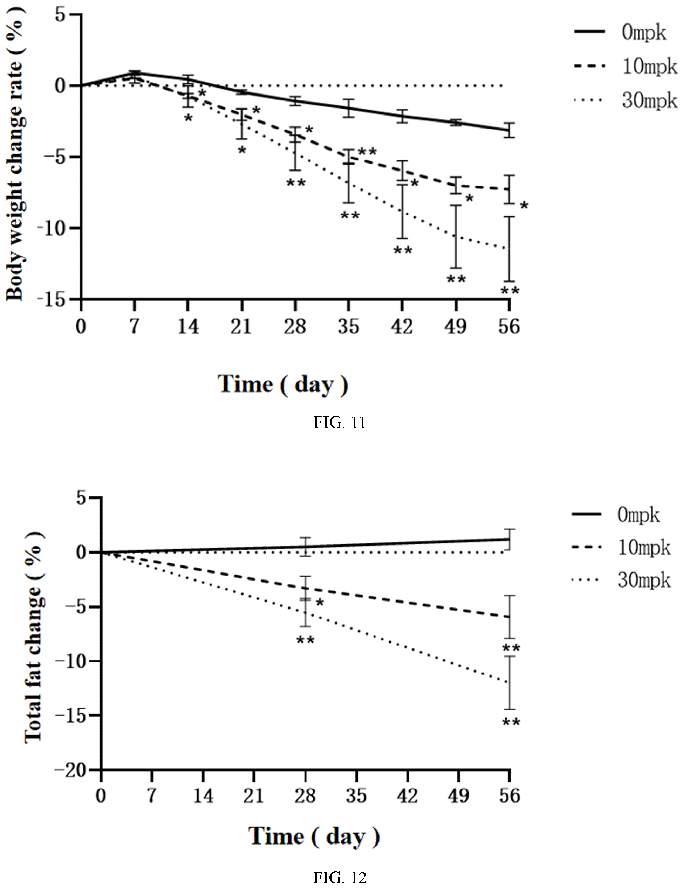

Diet-induced animal obesity models are similar to human obesity, and can better reflect the pathogenesis of human obesity. C57BL / 6J mice ( Beijing Huafukang Biotechnology Co., Ltd. ) are often used in diet-induced obesity ( DIO ) models because they are prone to developing severe obesity, lipogenesis accumulation, poor glucose tolerance, and moderate insulin resistance. In this example, male SPF C57BL / 6J mice aged 8 weeks and above and weighing 20-22 g were selected for modeling. The obese mouse model was induced by HFD ( 60 % high-fat diet ) ( model animal inclusion criteria : body weight 35-40 g at 16 weeks of HFD induction ). Obese mice were randomly assigned to the control group and the experimental group. The test doses were set to 10 mg / kg and 30 mg / kg, and the excipient control group was set up, and the excipient reference substance was given intravenously. Before and after administration ( weighing 8 times in total ), the body weight of the animals was weighed once a week, and the body weight of the mice was recorded ( FIG. 7 ). The change rate of body weight of mice was calculated according to the record of body weight of mice, and the change map of body weight of mice ( FIG. 8 ) was obtained. The change rate of body weight = ( current body weight-initial body weight ) / initial body weight × 100 %. The fat mass of mice was detected by DEXA, and the total fat change of mice was calculated to obtain the total fat change illustration of mice ( FIG. 9 ). Total fat change = ( current fat mass-initial fat mass ) / initial fat mass × 100 %. At the end of the experiment ( day 56 ), the results of the AB9 antibody on body weight, body weight change rate, and fat change in mice showed that in the diet-induced obesity model of mice, compared with the control group, the weight gain was inhibited in the anti-IGF-1R antibody administration group, and the fat content was significantly reduced. With the increase of the dose, the inhibition of weight gain and the decrease of fat content exhibited concentration dependence to some extent ( FIG. 10 - FIG. 12 ).

Finally, it should be noted that the above content is only used to illustrate the technical solution of the present invention, rather than to limit the protection scope of the present invention. Any minor modifications or equivalent substitutions made to the technical scheme of the present invention by a person skilled in the art do not depart from the essence and scope of the technical scheme of the present invention.

## Claims

1. Use of anti-IGF-1R antibody or antigen binding portion thereof in weight loss.

2. The use according to claim 1, **characterized in that** the weight loss is induced by anti-IGF-1R antibody or antigen binding portion thereof via at least one of the following mechanisms:
(1) inhibiting or blocking binding between IGF-1R and IGF-1;
(2) reducing, weakening or neutralizing IGF-1R activity;
(3) inhibiting cell proliferation;
(4) inhibiting the fat synthesis and/or accumulation of cells;
(5) inhibiting collagen synthesis of cells.

3. Use of anti-IGF-1R antibody or antigen binding portion thereof in the preparation of a product for preventing, treating obesity or obesity-related diseases.

4. The use according to claim 2, **characterized in that** the weight loss is induced by the product via at least one of the following mechanisms:
1) inhibiting or blocking binding between IGF-1R and IGF-1;
2) reducing, weakening or neutralizing IGF-1R activity;
3) inhibiting cell proliferation;
4) inhibiting the fat synthesis and/or accumulation of cells;
5) inhibiting collagen synthesis of cells.

5. The use according to claim 2 or 4, **characterized in that** the cells comprise fibroblasts.

6. The use according to claim 1 or 3, **characterized in that** the antibody or antigen binding portion thereof comprises a light chain variable region and a heavy chain variable region.

7. The use according to claim 6, **characterized in that** the light chain variable region has:
an LCDR1 as shown in SEQ ID NO : 25, SEQ ID NO : 31, SEQ ID NO : 37, SEQ ID NO : 43, SEQ ID NO : 49, SEQ ID NO : 55, SEQ ID NO : 61, SEQ ID NO : 67, SEQ ID NO : 73, SEQ ID NO: 79, SEQ ID NO : 85 or SEQ ID NO: 91 ; and
an LCDR2 as shown in SEQ ID NO : 26, SEQ ID NO : 32, SEQ ID NO : 38, SEQ ID NO : 44, SEQ ID NO : 50, SEQ ID NO : 56, SEQ ID NO : 62, SEQ ID NO : 68, SEQ ID NO : 74, SEQ ID NO : 80, SEQ ID NO : 86 or SEQ ID NO : 92 ; and
an LCDR3 as shown in SEQ ID NO : 27, SEQ ID NO : 33, SEQ ID NO : 39, SEQ ID NO : 45, SEQ ID NO : 51, SEQ ID NO : 57, SEQ ID NO : 63, SEQ ID NO : 69, SEQ ID NO : 75, SEQ ID NO: 81, SEQ ID NO : 87 or SEQ ID NO : 93.

8. The use according to claim 6, **characterized in that** the heavy chain variable region has:
an HCDR1 as shown in SEQ ID NO : 28, SEQ ID NO : 34, SEQ ID NO : 40, SEQ ID NO : 46, SEQ ID NO : 52, SEQ ID NO : 58, SEQ ID NO : 64, SEQ ID NO : 70, SEQ ID NO : 76, SEQ ID NO : 82, SEQ ID NO : 88 or SEQ ID NO : 94 ; and
an HCDR2 as shown in SEQ ID NO : 29, SEQ ID NO : 35, SEQ ID NO : 41, SEQ ID NO : 47, SEQ ID NO : 53, SEQ ID NO : 59, SEQ ID NO : 65, SEQ ID NO : 71, SEQ ID NO : 77, SEQ ID NO : 83, SEQ ID NO : 89 or SEQ ID NO : 95 ; and
an HCDR3 as shown in SEQ ID NO : 30, SEQ ID NO : 36, SEQ ID NO : 42, SEQ ID NO : 48, SEQ ID NO : 54, SEQ ID NO : 60, SEQ ID NO : 66, SEQ ID NO : 72, SEQ ID NO : 78, SEQ ID NO : 84, SEQ ID NO : 90 or SEQ ID NO : 96.

9. The use according to claim 7 or 8, **characterized in that** the light chain of the antibody or the antigen binding portion is formed by splicing a light chain variable region and a light chain constant region as shown in SEQ ID NO : 97; and the heavy chain of the antibody or the antigen binding portion is formed by splicing a heavy chain variable region and a heavy chain constant region as shown in any one of SEQ ID NO : 98-104.

10. The use according to claim 6, **characterized in that** the light chain variable region has:
an amino acid sequence as shown in SEQ ID NO : 1, SEQ ID NO : 3, SEQ ID NO : 5, SEQ ID NO : 7, SEQ ID NO : 9, SEQ ID NO: 11, SEQ ID NO: 13, SEQ ID NO: 15, SEQ ID NO : 17, SEQ ID NO: 19, SEQ ID NO : 21 or SEQ ID NO : 23.

11. The use according to claim 6, **characterized in that** the heavy chain variable region has:
an amino acid sequence as shown in SEQ ID NO : 2, SEQ ID NO : 4, SEQ ID NO : 6, SEQ ID NO : 8, SEQ ID NO : 10, SEQ ID NO : 12, SEQ ID NO : 14, SEQ ID NO : 16, SEQ ID NO : 18, SEQ ID NO : 20, SEQ ID NO : 22 or SEQ ID NO : 24.

12. The use according to claim 7, **characterized in that** the light chain variable region has:
the LCDR1 as shown in SEQ ID NO : 25, the LCDR2 as shown in SEQ ID NO : 26, and the LCDR3 as shown in SEQ ID NO : 27;
or, the LCDR1 as shown in SEQ ID NO : 31, the LCDR2 as shown in SEQ ID NO : 32, and the LCDR3 as shown in SEQ ID NO : 33;
or, the LCDR1 as shown in SEQ ID NO : 37, the LCDR2 as shown in SEQ ID NO : 38, and the LCDR3 as shown in SEQ ID NO : 39;
or, the LCDR1 as shown in SEQ ID NO : 43, the LCDR2 as shown in SEQ ID NO : 44, and the LCDR3 as shown in SEQ ID NO : 45;
or, the LCDR1 as shown in SEQ ID NO : 49, the LCDR2 as shown in SEQ ID NO : 50, and the LCDR3 as shown in SEQ ID NO : 51;
or, the LCDR1 as shown in SEQ ID NO : 55, the LCDR2 as shown in SEQ ID NO : 56, and the LCDR3 as shown in SEQ ID NO : 57;
or, the LCDR1 as shown in SEQ ID NO : 61, the LCDR2 as shown in SEQ ID NO : 62, and the LCDR3 as shown in SEQ ID NO : 63;
or, the LCDR1 as shown in SEQ ID NO : 67, the LCDR2 as shown in SEQ ID NO : 68, and the LCDR3 as shown in SEQ ID NO : 69;
or, the LCDR1 as shown in SEQ ID NO : 73, the LCDR2 as shown in SEQ ID NO : 74, and the LCDR3 as shown in SEQ ID NO : 75;
or, the LCDR1 as shown in SEQ ID NO : 79, the LCDR2 as shown in SEQ ID NO : 80, and the LCDR3 as shown in SEQ ID NO : 81;
or, the LCDR1 as shown in SEQ ID NO : 85, the LCDR2 as shown in SEQ ID NO : 86, and LCDR3 as shown in SEQ ID NO : 87;
or, the LCDR1 as shown in SEQ ID NO : 91, the LCDR2 as shown in SEQ ID NO : 92, and LCDR3 as shown in SEQ ID NO : 93.

13. The use according to claim 7, **characterized in that** the heavy chain variable region has:
the HCDR1 as shown in SEQ ID NO : 28, the HCDR2 as shown in SEQ ID NO : 29, the and HCDR3 as shown in SEQ ID NO : 30;
or, the HCDR1 as shown in SEQ ID NO : 34, the HCDR2 as shown in SEQ ID NO: 35, the and HCDR3 as shown in SEQ ID NO : 36;
or, the HCDR1 as shown in SEQ ID NO: 40, the HCDR2 as shown in SEQ ID NO : 41, the and HCDR3 as shown in SEQ ID NO : 42;
or, the HCDR1 as shown in SEQ ID NO : 46, the HCDR2 as shown in SEQ ID NO : 47, the and HCDR3 as shown in SEQ ID NO : 48;
or, the HCDR1 as shown in SEQ ID NO: 52, the HCDR2 as shown in SEQ ID NO : 53, the and HCDR3 as shown in SEQ ID NO : 54;
or, the HCDR1 as shown in SEQ ID NO: 58, the HCDR2 as shown in SEQ ID NO : 59, the and HCDR3 as shown in SEQ ID NO : 60;
or, the HCDR1 as shown in SEQ ID NO : 64, the HCDR2 as shown in SEQ ID NO : 65, the and HCDR3 as shown in SEQ ID NO : 66;
or, the HCDR1 as shown in SEQ ID NO : 70, the HCDR2 as shown in SEQ ID NO : 71, the and HCDR3 as shown in SEQ ID NO : 72;
or, the HCDR1 as shown in SEQ ID NO : 76, the HCDR2 as shown in SEQ ID NO : 77, the and HCDR3 as shown in SEQ ID NO : 78;
or, the HCDR1 as shown in SEQ ID NO : 82, the HCDR2 as shown in SEQ ID NO : 83, the and HCDR3 as shown in SEQ ID NO : 84;
or, the HCDR1 as shown in SEQ ID NO : 88, the HCDR2 as shown in SEQ ID NO : 89, the and HCDR3 as shown in SEQ ID NO : 90;
or, the HCDR1 as shown in SEQ ID NO : 94, the HCDR2 as shown in SEQ ID NO : 95, the and HCDR3 as shown in SEQ ID NO : 96.

14. The use according to claim 3, **characterized in that** the obesity-related diseases comprise liver steatosis, hypertension, hyperglycemia, and high cholesterol.

15. The use according to claim 3, **characterized in that** the product comprises medicine, health care products and food.

16. The use according to claim 15, **characterized in that** the product comprises the antibody or the antigen binding portion, or a biological material comprising the antibody or the antigen binding portion or a gene thereof, and at least one pharmaceutically acceptable excipient, where the biological material comprises expression vectors and host cells.

17. The use according to claim 16, **characterized in that** the excipient is selected from solvents, diluents, disintegrants, precipitation inhibitors, surfactants, glidants, binders, lubricants, dispersants, suspending agents, isotonic agents, thickeners, emulsifiers, preservatives, stabilizers, hydrating agents, emulsifying accelerators, buffers, absorbers, colorants, scents, sweeteners, ion exchangers, release agents, coating agents, flavoring agents or antioxidants.
